# EUROPEAN PATENT APPLICATION

(11) **EP 2 415 770 A1**
(43) Date of publication of application: **08.02.2012**
(21) Application number: 10758036.7
(22) Date of filing: 29.03.2010
(51) Int. Cl.: C07D 471/04, A61K 31/435, A61P 25/00, A61P 25/16, A61P 25/28

(54) **LITHIUM DERIVATIVES OF PYRROLOQUINOLINE QUINONE AND PREPARATION METHOD THEREOF**

(30) Priority: 03.04.2009 CN 200910048873
(71) Applicant: Shanghai Rixin Bio-techonology Co., Ltd., Shanghai 200031 (CN)
(72) Inventor: ZHONG, Chunjiu, Shanghai 200031 (CN); YANG, Qing, Shanghai 200031 (CN)
(74) Representative: Michalski Hüttermann & Partner Patentanwälte
(86) International application number: PCT/CN2010/071382
(87) International publication number: WO 2010/111934

(57) **Abstract**

Lithium derivatives of pyrroloquinoline quinone and preparation method thereof are disclosed. In said method, lithium derivatives of pyrroloquinoline quinone are obtained by an acid-base neutralization reaction in a basic solvent with pyrroloquinoline quinine (PQQ) as a starting material. Lithium ion is brought in the molecular structure of pyrroloquinoline quinone to form said lithium derivatives of pyrroloquinoline quinone. The reaction condition of said method is mild, the product is easy to be purified, the preparation procedure is simple, and the yield is more than 80 %. Said lithium derivatives of pyrroloquinoline quinone possess GSK-3 inhibiting activity and possess the functions such as reducing the formation of age pigment in the brain of a transgenic mouse and reducing the phosphorylation of tau proteins. Said lithium derivatives of pyrroloquinoline quinone may be used in the manufacture of medicaments for preventing and treating senile dementia, senility or Parkinson's disease.

## Description

### Technical Field

The present invention belongs to the pharmaceutical field, and relates to lithium derivatives of pyrroloquinoline-quinone and the preparation method thereof.

### Background of the invention

As reported on Nature, 2003, (Nature 2003; 422: 832), Japanese scientists discovered that pyrroloquinoline-quinone (PQQ), a new B vitamin, functions in Lysine metabolism in vivo. PQQ was first discovered in micro-organisms and also exists in higher eukaryotic organisms. Existing technologies have disclosed that the molecular weight of PQQ is 330, and have identified the crystal structure and its chemical synthesis. The formula of PQQ is as follow:

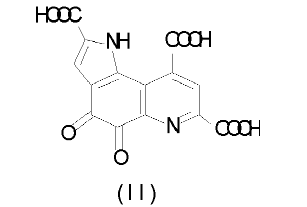

PQQ is a cofactor for many important enzymes and can affect the respiratory chain function and free radicals level in vivo. Studies show that mice lacking PQQ grow slowly, have problems in reproducing, and prone to suffer arthritis, so that PQQ is considered as necessary vitamins and nutrients in vivo. Functions of PQQ related to the nervous system have been shown in the following four aspects: 1) PQQ is an anti-oxidant and free radical scavenger. 2) PQQ has an effect on the respiratory chain function and can protect mitochondrial energy metabolism. 3) PQQ can stimulate the secretion of nerve growth factor, protect nerve and promote its growth. 4) PQQ can slow the deposition of α-synuclein protein and protect nerve cells from fibrosis. These researches suggest potential therapeutic value of PQQ for Parkinson's disease, senile dementia and other neurodegenerative diseases.

Lithium salts is the first found inhibitor of Glycoden synthase kinase-3 (GSK-3). Studies have shown that Lithium salts have potential therapeutic value for a variety of neurological and psychiatric disorders. Therefore, researchers pay attention to potential value of lithium derivatives of pyrroloquinoline quinone and attempt to figure out the possibility and mechanism of lithium derivatives of pyrroloquinoline quinone to treat neurological and psychiatric disorders.

### Summary of the invention

One object of the present invention is to provide a compound of lithium derivatives of pyrroloquinoline quinone.

Another object of the present invention is to provide the preparation method of the compound of lithium derivatives of pyrroloquinoline quinone. The invention conducts derivatization reaction by introducing lithium ion into PQQ, and obtains lithium derivatives of pyrroloquinoline quinone. Specifically, the invention conducts acid-base neutralization reaction with carboxylic acid groups of PQQ.

The present invention uses PQQ of formula (II) as raw material, conducts acid-base neutralization reaction in the alkaline solution of sodium hydroxide, and obtains lithium derivatives of pyrroloquinoline quinone of formula (I). Among R1, R2 and R3, at least one represent lithium ion.

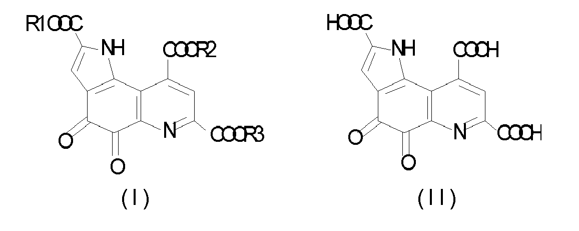

The present invention introduce lithium ion in the molecular structure of pyrroloquinoline quinone, with mild reaction conditions, easily refined and purified product, simple process and a high yield of over 80%, which is good for industrial production. Reaction expressed as follows, 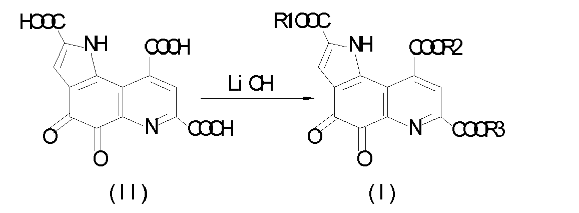 wherein R1, R2 and R3 is individually selected from hydrogen, ammonium ion (NH3), potassium, sodium, magnesium, calcium, zinc ion and lithium ion and at least one is lithium ion.

In the present invention, the addition of acid or base catalyst is not necessary in the said reaction, while lithium hydroxide itself could control the hydrogen potential (PH) value in the neutralization reaction, since varied concentration of lithium hydroxide had different basic strength and could affect the yeild of the compound of formula (I).

In the present invention, the said reaction could be carried out at the temperture of from 0 to 100°C, preferably from 15 to 20°C. The reaction time is the range of 15 minutes to 72 hours since each kind of lithium derivatives of pyrroloquinoline-quinone needs different time.

In the present invention, lithium derivatives of pyrroloquinoline quinone of formula (I) are made into lithium salts by the conventional methods of salt formation in basic solvent.

A further object of the present invention is the provision of usefulness of lithium derivatives of pyrroloquinoline-quinone of formula (I) in the manufacture of medicaments for preventing and treating senile dementia, senility or Parkinson's disease.

The present invention carries out studies on the use of lithium derivatives of pyrroloquinoline-quinone obtained on inhibiting the activity of GSK-3 and treating Alzheimer's disease. Studies show that said lithium derivatives of pyrroloquinoline-quinone possess functions such as inhibiting the activity of GSK-3 and reducing the formation of senile plaques in the brains of transgenic mice and phosphorylation of tau proteins. Said lithium derivatives of pyrroloquinoline-quinone can be used in the manufacture of medicaments for preventing and treating senile dementia, senility or Parkinson's disease.

Medicaments according to the invention include the following: Tablets, powders, powder injection, rectal Suppositories, skin pateches, water injection and sprays. Compounds of the present invention can be administered to human orally, intramuscularly, intraperitoneally, intravenously, nasally or rectally. The daily dose is 0.1-1000 mg for preventing and treating diseases such as Alzheimer's disease, senile dementia and senility.

To facilitate a better understanding, the present invention is further illustrated by the following examples. It should be understood that these examples are illustrative only and will not intend to limit the scope of the invention. Obviously, the skilled in the art could make various changes or modifications to the invention, and these equivalents would still be within the scope of the present invention.

### Brief description of the drawings

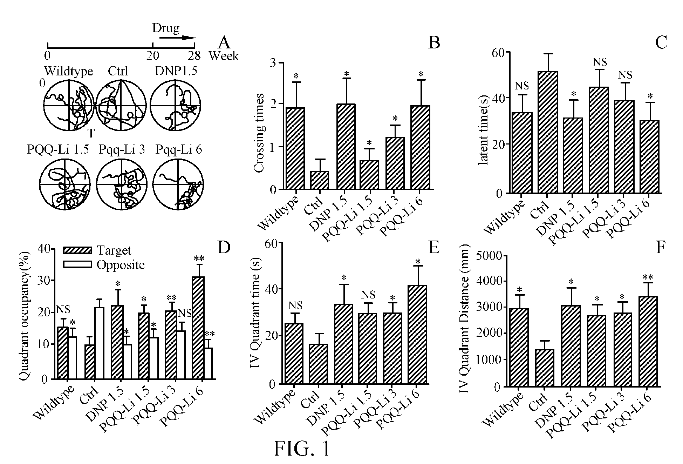

Fig. 1 shows that lithium pyrroloquinoline-quinone promotes the cognitive ability of APP/PSI transgenic mice.

### Detailed description of the embodiments of the invention

In the following embodiments, the temperture is expressed in degrees Celsius (°C).

### Example 1: Synthesis of 4,5-dihydroxy-1H-pyrrole[2,3-f]chinoline-2,7,9-tricarboxylic acid, trilithium salt (PQQ3Li):

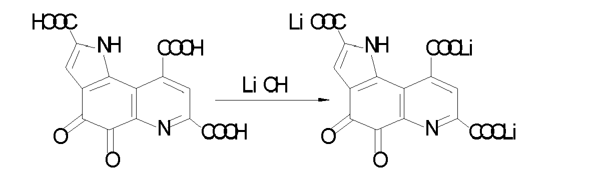

To a 1L reaction kettle, 15 g of pyrroloquinoline-quinone (PQQ) and 450 ml of tetrahydrofuran(THF) were added. With the solution being stirred, 5.9 g of lithium hydroxide monohydrate dissolved in 150 ml of water were added dropwise. The mixture was then stirred at the temperature of 15-20 °C for 24 hours. Hydrochloric acid was added to neutralize the reaction and a red-brown solid is precipitated, which was separated by filtration to obtain 14.3 g red-brown powder of PQQ3Li with a yield of 90.5%.

### Example 2: Synthesis of 4,5-dihydroxy-1H-pyrrole[2,3-f]chinoline-2,7,9-tricarboxylic acid, dilithium salt (PQQ2Li):

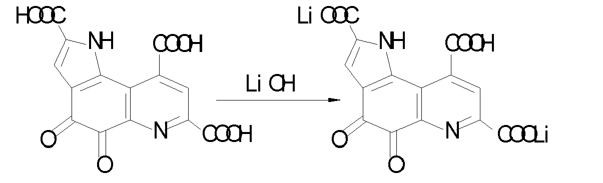

To a 1L reaction kettle, 15 g of pyrroloquinoline-quinone (PQQ) and 450 ml of tetrahydrofuran (THF) were added. With the solution being stirred, 3.93 g of lithium hydroxide monohydrate dissolved in 150 ml of water were added dropwise. The mixture was then stirred at the temperature of 15-20 °C for 24 hours. Hydrochloric acid was added to neutralize the mixture and a red-brown solid is precipitated, which was separated by filtration to obtain 13.0 g red-brown powder of PQQ2Li with a yield of 83.9%.

### Example 3: Synthesis of 4,5-dihydroxy-1H-pyrrole[2,3-f]chinoline-2,7,9-tricarboxylic acid, lithium salt (PQQLi):

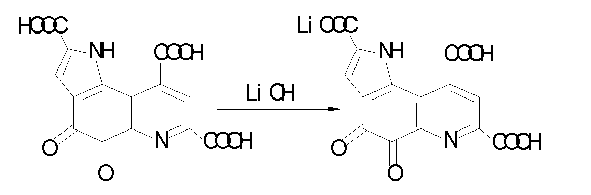

To a 1L reaction kettle, 15 g of pyrroloquinoline-quinone (PQQ) and 450 ml of tetrahydrofuran (THF) were added. With the solution being stirred, 1.96 g of lithium hydroxide monohydrate dissolved in 150 ml of water were added dropwise. The mixture was then stirred at the temperature of 15-20 °C for 24 hours. Hydrochloric acid was added to neutralize the mixture and a red-brown solid is precipitated, which was separated by filtration to obtain 8.1 g red-brown powder of PQQ2Li with a yield of 83.9%.

### Example 4: Test of the use of the compounds in treating senile dementia

### 1. Preparation of different types of lithium derivatives of pyrroloquinoline-quinone:

According to conventional methods, medicaments of said compounds were prepared in the following forms: Tablets, powders, powder injection, rectal suppositories, skin pateches, water injection and sprays.

### 2. Clinical experiments for different types of lithium derivatives of pyrroloquinoline-quinone:

Different types of said compounds were administered orally, intramuscularly, intraperitoneally, intravenously, nasally or rectally. The daily dose was 0.1∼1000 mg for preventing or treating Alzheimer's disease or senility.

### 3. Drug test on mice:

Kunming mice of naturally aging and Alzheimer's transgenic mice were orally or intraperitoneally administrated with lithium salts of pyrroloquinoline-quinone for two consecutive months, with a dosage of 1 mg/kg/day. Untreated mice were used as a control group. The following results have been obtained: Treated mice showed significant improvement in cognitive function and extension of average life expectancy. It suggested the use of lithium derivatives of pyrroloquinoline quinone for preventing or treating Alzheimer's disease or senility.

Same kinds of mice were administrated with a mixture of drugs for two months (continuously or consecutively) with a dosage of 0.1-1000 mg/day. Said mixture of drugs comprises lithium derivatives of pyrroloquinoline quinone, benfotiamine and/or coenzyme Q10. Dosage of lithium salts of pyrroloquinoline quinone was 0.1-1000 mg/day and that of benfotiamine and coenzyme Q10 were sperately 1-1000 mg/day. Studies suggested that combinition of lithium salts of pyrroloquinoline quinone, benfotiamine and/or coenzyme Q10 is also useful in preventing or treating Alzheimer's disease or senility.

### Example 5: Test of the use of the compounds in treating Parkinson's disease

### 1. Preparation of different types of lithium derivatives of pyrroloquinoline-quinone:

According to conventional methods, medicaments of said compounds were prepared in the following forms: Tablets, powders, powder injection, rectal suppositories, skin pateches, water injection and sprays.

### 2. Clinical experiments for different types of lithium derivatives of pyrroloquinoline-quinone:

Different types of said compounds were administered orally, intramuscularly, intraperitoneally, intravenously, nasally or rectally. The daily dose was 0.1∼1000 mg for preventing or treating Parkinson's disease.

### 3. Drug test on rats:

Parkinson's disease model rats induced by 6-hydroxydopamine were orally or intraperitoneally administrated with lithium salts of pyrroloquinoline-quinone for two months (continuously or consecutively), with a dosage of 1 mg/kg/day. Untreated mice were used as a control group. The following results have been obtained: Treated mice showed significant improvement in movement function. It suggested the use of lithium derivatives of pyrroloquinoline quinone for preventing or treating Parkinson's disease.

Same kinds of mice were administrated with a mixture of drugs for two consecutive months with a dosage of 0.1-1000 mg/day. Said mixture of drugs comprises lithium derivatives of pyrroloquinoline quinone, benfotiamine and/or coenzyme Q10. Dosage of lithium salts of pyrroloquinoline quinone was 0.1-1000 mg/day and that of benfotiamine and coenzyme Q10 were sperately 1-1000 mg/day. Studies suggested that combinition of lithium salts of pyrroloquinoline quinone, benfotiamine and/or coenzyme Q10 is also useful in preventing or treating Parkinson's disease.

Fig. 1 shows that lithium pyrroloquinoline-quinone promotes the cognitive ability of APP/PSI transgenic mice. Intragastric administration is applied to 20-week-old mice. Wiletype is a wiletype control group; Ctrl is a transgenic control group; DNP is a dinonyl phthalate group (1.5 mg/kg of weight); PQQLi 1.5 is a lithium pyrroloquinoline-quinone salt group (1.5 mg/kg of weight); PQQLi 6 is a lithium pyrroloquinoline-quinone group (6 mg/kg of weight). *p < 0.05;
** p < 0.005; NS: p < 0.05.

## Claims

1. The salts of lithium derivatives of pyrroloquinoline-quinone with formulae (I), 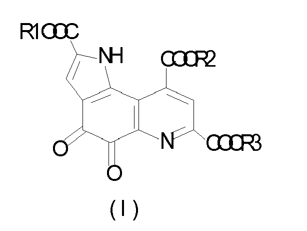 wherein R1, R2 and R3, is individually selected from hydrogen, ammonium ion (NH3), potassium, sodium, magnesium, calcium, zinc ion and lithium ion, and at least one of the three is lithium ion.

2. A preparation method of said lithium derivatives of pyrroloquinoline-quinone as defined by claim 1, which comprises an acid-base neutralization reaction as follow, wherein the compound of formula (II) reacting as a staring material within lithium hydroxide solution, and converting into said lithium derivatives of pyrroloquinoline-quinone of formula (I).

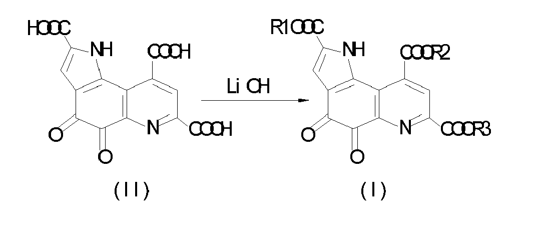

3. The preparation method as defined by claim 2, wherein said basic solvent is lithium hydroxide solution.

4. The preparation method as defined by claim 2, wherein reactions don't need catalyst.

5. The preparation method as defined by claim 2, wherein the temperature is in the range of 0°C to 100°C.

6. The preparation method s as defined by claim 2, wherein the temperature is about 15-20°C.

7. The preparation method as defined by claim 2, wherein the reaction time is the range of 15 minutes to 72 hours.

8. The use of lithium derivatives of pyrroloquinoline-quinone as defined by claim 1 in the manufacture of medicaments for preventing and treating senile dementia, senility or Parkinson's disease.
